# EUROPEAN PATENT APPLICATION

(11) **EP 3 682 801 A1**
(43) Date of publication of application: **22.07.2020**
(21) Application number: 18857288.7
(22) Date of filing: 14.09.2018
(51) Int. Cl.: A61B 5/107

(54) **EVALUATION METHOD, SELECTION METHOD, EVALUATION ASSISTANCE TOOL, AND SCREENING METHOD**

(30) Priority: 15.09.2017 JP 2017178044; 13.06.2018 JP 2018113115
(71) Applicant: Milbon Co., Ltd., Osaka 534-0015 (JP)
(72) Inventor: WATANABE, Kosuke, Osaka-shi Osaka 534-0015 (JP); NISHIDA, Momoko, Osaka-shi Osaka 534-0015 (JP); YAMADA, Makiko, Osaka-shi Osaka 534-0015 (JP); NAGAMI, Keiko, Osaka-shi Osaka 534-0015 (JP); SAKURAI, Yuuki, Osaka-shi Osaka 534-0015 (JP)
(74) Representative: Global IP Europe Patentanwaltskanzlei
(86) International application number: PCT/JP2018/034227
(87) International publication number: WO 2019/054494

(57) **Abstract**

The present invention provides at least any one of a novel evaluation method, a selection method, an evaluation support tool and a screening method for a scalp and/or hair. The evaluation method, the selection method, the evaluation support tool and the screening method are employed for evaluation of at least any one of scalp redness, an index related to the redness, a largeness in number of gray hairs, a smallness in number of hairs and a largeness of variation in hair thickness of an evaluatee based on information on bacteria of the genus Corynebacterium present in a scalp flora of the evaluatee, for evaluation of information on bacteria of the genus Corynebacterium present in a scalp flora of an evaluatee based on at least any one of scalp redness, an index related to the redness, a largeness in number of gray hairs, a smallness in number of hairs and a largeness of variation in hair thickness of the evaluatee, or for evaluation of at least any one of a largeness in number of gray hairs, a smallness in number of hairs, a largeness of variation in hair thickness and a thin hair ratio according to scalp redness or an index related to the redness of an evaluatee.

## Description

### TECHNICAL FIELD

The present invention relates to an evaluation method, a selection method, an evaluation support tool and a screening method.

### BACKGROUND ART

Hair damage and the like have been conventionally visually diagnosed.

On the contrary, Patent Literature 1 proposes, for purposes of more appropriately diagnosing hair damage, a diagnostic method in which enlarged images of a plurality of portions of hair of a subject are simultaneously displayed on a diagnosis screen for making a diagnosis through comparison between a normal portion and a damaged portion of the hair.

### SUMMARY OF THE INVENTION

### <Technical Problem>

All conventional evaluation methods for hair including the diagnostic method described in Patent Literature 1 are methods in which the condition of hair is evaluated by visually checking the condition of the hair itself or comparing images of hair itself, and an evaluation method for hair different from the methods for checking the condition of hair itself has been neither proposed nor examined. Besides, any evaluation method for a scalp has not been proposed so far.

The present disclosure is given in consideration of the aforementioned circumstances, and an object is to provide at least any one of a novel evaluation method, a selection method, an evaluation support tool and a screening method for a scalp and/or hair.

### <Solution to Problem>

### (Disclosure 1)

The present inventors have made earnest studies on the influence, on a scalp and/or hair, of bacteria present in a scalp flora, which has never been noticed before, resulting in finding that the presence of bacteria of the genus Corynebacterium in the scalp flora of an evaluatee is related to scalp redness, a largeness in number of gray hairs, a smallness in number of hairs and a largeness of variation in hair thickness. The inventors have conceived to use this relationship as an evaluation method for a scalp and/or hair, and thus, present disclosure 1 was accomplished.

Specifically, in an evaluation method of the present disclosure 1, at least any one of scalp redness, an index related to the redness, the largeness in number of gray hairs, the smallness in number of hairs and the largeness of variation in hair thickness of an evaluatee is evaluated based on information on bacteria of the genus Corynebacterium present in a scalp flora of the evaluatee, or information on bacteria of the genus Corynebacterium present in a scalp flora of an evaluatee is evaluated based on at least any one of scalp redness, an index related to the redness, the largeness in number of gray hairs, the smallness in number of hairs and the largeness of variation in hair thickness of the evaluatee.

In this evaluation method, the information on bacteria of the genus Corynebacterium present in the scalp flora of the evaluatee may include an abundance ratio of bacteria of the genus Corynebacterium in the scalp flora of the evaluatee, and the information on bacteria of the genus Corynebacterium of the scalp flora of the evaluatee may consist of merely the abundance ratio of bacteria of the genus Corynebacterium in the scalp flora of the evaluatee.

In particular, evaluation is performed, in the evaluation method, preferably by using a comparison criterion for the abundance ratio of bacteria of the genus Corynebacterium in a scalp flora.

Besides, in the evaluation method, the evaluation is performed preferably by using a comparison criterion, according to an age of the evaluatee, for the abundance ratio of bacteria of the genus Corynebacterium in the scalp flora. Thus, more accurate evaluation can be performed by this evaluation method.

Besides, in a selection method for a scalp/hair cosmetic according to the disclosure 1, a scalp/hair cosmetic to be recommended to an evaluatee is selected according to an evaluation result obtained by the above-described evaluation method. Since the information on bacteria of the genus Corynebacterium of the evaluatee is thus used, a scalp/hair cosmetic suitable to the evaluatee can be selected.

In the selection method for a scalp/hair cosmetic, a scalp/hair cosmetic containing an anti-inflammatory agent is preferably selected based on the evaluation result. According to inflammatory condition of the scalp found based on the information on bacteria of the genus Corynebacterium of the evaluatee, a scalp/hair cosmetic suitable to the evaluatee can be selected.

Besides, an evaluation support tool according to the disclosure 1 is an evaluation support tool for supporting evaluation of at least any one of scalp redness, an index related to the redness, a largeness in number of gray hairs, a smallness in number of hairs and a largeness of variation in hair thickness of an evaluatee based on information on bacteria of the genus Corynebacterium present in a scalp flora of the evaluatee, or evaluation of information on bacteria of the genus Corynebacterium present in a scalp flora of an evaluatee based on at least any one of scalp redness, an index related to the redness, a largeness in number of gray hairs, a smallness in number of hairs and a largeness of variation in hair thickness of the evaluatee. The evaluation support tool displays a comparison criterion for an abundance ratio of bacteria of the genus Corynebacterium present in a scalp flora.

Since the comparison criterion for an abundance ratio of bacteria of the genus Corynebacterium in a scalp flora can be displayed in the evaluation support tool so as to be compared with obtained information on bacteria of the genus Corynebacterium present in a scalp flora of the evaluatee, the evaluation of at least any one of the scalp redness, the index related to the redness, the largeness in number of gray hairs, the smallness in number of hairs and the largeness of variation in hair thickness of the evaluatee is supported. Besides, since the comparison criterion for an abundance ratio of bacteria of the genus Corynebacterium in the scalp flora is displayed in the evaluation support tool, when the abundance ratio of bacteria of the genus Corynebacterium in the scalp flora of the evaluatee is to be evaluated based on at least any one of the scalp redness, the index related to the redness, the largeness in number of gray hairs, the smallness in number of hairs and the largeness of variation in hair thickness of the evaluatee, evaluation of whether or not the abundance ratio of the evaluatee is higher than the comparison criterion can be supported.

Furthermore, a screening method for a scalp/hair cosmetic according to the disclosure 1 includes the steps of: treating a scalp and/or hair of an evaluatee at least once by using a scalp/hair cosmetic; and comparing abundance ratios of bacteria of the genus Corynebacterium in a scalp flora of the evaluatee before and after the step of treating a scalp and/or hair. When this screening method is employed, a scalp/hair cosmetic capable of lowering the abundance ratio of bacteria of the genus Corynebacterium in the scalp flora of the evaluatee can be easily selected.

A screening method for a candidate substance for an active ingredient according to the disclosure 1 includes the steps of: treating a scalp and/or hair of an evaluatee with a candidate substance for an active ingredient at least once; and comparing abundance ratios of bacteria of the genus Corynebacterium in a scalp flora of the evaluatee before and after the step of treating a scalp and/or hair. When this screening method is employed, a candidate substance for an active ingredient capable of lowering the abundance ratio of bacteria of the genus Corynebacterium in the scalp flora of the evaluatee can be easily selected.

Besides, a screening method for selecting an evaluatee according to the disclosure 1 includes the steps of: checking an abundance ratio of bacteria of the genus Corynebacterium in a scalp flora of an evaluatee; and comparing the abundance ratio of the evaluatee with a comparison criterion for an abundance ratio of bacteria of the genus Corynebacterium in a scalp flora. When this screening method is employed, a person having an abundance ratio of bacteria of the genus Corynebacterium higher than the comparison criterion and a person having an abundance ratio lower than the comparison criterion can be easily sorted out.

### (Disclosure 2)

In addition, the present inventors have made earnest studies from a viewpoint of the influence of scalp redness on hair, which viewpoint has not been conventionally considered, resulting in newly finding that a largeness in number of gray hairs, a smallness in number of hairs, a largeness of variation in hair thickness and a thin hair ratio are varied depending on scalp redness of an evaluatee. The inventors have further found that the largeness in number of gray hairs, the smallness in number of hairs, the largeness of variation in hair thickness and a thin hair ratio of an evaluatee can be diagnosed by paying attention to scalp redness of the evaluatee, and thus, present disclosure 2 was accomplished.

Specifically, in an evaluation method according to the disclosure 2, at least any one of the largeness in number of gray hairs, the smallness in number of hairs, the largeness of variation in hair thickness and a thin hair ratio is evaluated according to scalp redness or an index related to the redness of an evaluatee.

In the evaluation method, evaluation is performed preferably by using information corresponding to a comparison criterion, according to an age of the evaluatee, for the scalp redness or the index related to the redness.

Although a proportion of persons having scalp redness and the degree of the redness are varied according to the age, when this evaluation method is employed, positioning, in persons of the same age group as the evaluatee, of the condition of the scalp of the evaluatee can be objectively found. Besides, when the evaluation is performed by using a plurality of information of a plurality of age groups, at least any one of the largeness in number of gray hairs, the smallness in number of hairs, the largeness of variation in hair thickness and the thin hair ratio in the future can be predicted assuming that the evaluatee gets older with the current scalp redness or the like kept, or assuming that the current scalp redness or the like are changed through aging of the evaluatee.

Besides, in a selection method for a hair treatment agent according to the disclosure 2, a hair treatment agent to be recommended to an evaluatee is selected according to an evaluation result obtained by the above-described evaluation method.

In the selection method for a hair treatment agent, a hair treatment agent containing an anti-inflammatory agent is preferably selected based on the evaluation result. Besides, in the selection method for a hair treatment agent, a scalp/hair cosmetic is preferably selected because such a cosmetic can be easily caused to affect not only the hair but also the scalp particularly.

An evaluation support tool according to the disclosure 2 is an evaluation support tool for supporting evaluation of any one of a largeness in number of gray hairs, a smallness in number of hairs, a largeness of variation in hair thickness and a thin hair ratio, and displays information corresponding to a comparison criterion to be compared with scalp redness or an index related to the redness of an evaluatee.

### <Advantageous Effects of Invention>

According to the evaluation method of the disclosure 1, a novel evaluation method for a scalp and/or hair is provided.

According to the selection method for a scalp/hair cosmetic of the disclosure 1, a scalp/hair cosmetic suitable to an evaluatee can be selected.

According to the evaluation support tool of the disclosure 1, the novel evaluation method is supported.

According to the screening method for a scalp/hair cosmetic of the disclosure 1, a scalp/hair cosmetic capable of lowering an abundance ratio of bacteria of the genus Corynebacterium in a scalp flora of an evaluatee is easily selected.

According to the screening method for a candidate substance for an active ingredient of the disclosure 1, a candidate substance for an active ingredient capable of lowering an abundance ratio of bacteria of the genus Corynebacterium in a scalp flora of an evaluatee is easily selected.

According to the screening method for an evaluatee of the disclosure 1, a person having an abundance ratio of bacteria of the genus Corynebacterium higher than a comparison criterion and a person having an abundance ratio lower than the comparison criterion are easily sorted out.

According to the evaluation method, the selection method for a hair treatment agent and the evaluation support tool of the disclosure 2, unprecedented and novel evaluation can be performed on at least any one of the largeness in number of gray hairs, the smallness in number of hairs, the largeness of variation in hair thickness and a thin hair ratio.

### BRIEF DESCRIPTION OF THE DRAWINGS

Figure 1 illustrates analysis results of scalp floras of a group having a high degree of scalp redness and a group having a low degree of scalp redness of persons in their 30s.
Figure 2 illustrates analysis results of scalp floras of a group having a high degree of scalp redness and a group having a low degree of scalp redness of persons in their 50s.
Figure 3 illustrates a table showing images of scalps according to deviations of an a* value and age groups.
Figure 4 illustrates a table showing groups having different deviations of the a* value of the scalp according to age groups.
Figure 5 is a table showing images of scalps of persons in their 20s separately as a group having high a* values and a group having low a* values.
Figure 6 is a table showing images of scalps of persons in their 30s separately as a group having high a* values and a group having low a* values.
Figure 7 is a table showing images of scalps of persons in their 40s separately as a group having high a* values and a group having low a* values.
Figure 8 is a table showing images of scalps of persons in their 50s separately as a group having high a* values and a group having low a* values.
Figure 9 is a table showing images of scalps of persons in their 60s separately as a group having high a* values and a group having low a* values.
Figure 10 is a graph illustrating gray hair ratios according to age groups separately as a group having high a* values and a group having low a* values.
Figure 11 is a graph illustrating a total number of hairs in unit area according to the age groups separately as a group having high a* values and a group having low a* values.
Figure 12 is a graph illustrating standard deviations of hair diameters according to the age groups separately as a group having high a* values and a group having low a* values.

### DESCRIPTION OF EMBODIMENTS

Now, an evaluation method, a selection method, an evaluation support tool and a screening method according to embodiments of the present disclosure will be described by way of examples, and it is noted that the disclosure contents are not limited to these examples. It is noted that the term "and/or" as used herein means both or either.

### (1) Evaluation Method

### (1-1) Evaluation Method of Embodiment 1

An evaluation method of Embodiment 1 is a method for evaluating, based on information on bacteria of the genus Corynebacterium present in a scalp flora of an evaluatee, at least any one of scalp redness, an index related to the redness (hereinafter the "redness or the index related to the redness" are sometimes referred to as the "redness or the like"), a largeness in number of gray hairs, a smallness in number of hairs, and a largeness of variation in hair thickness of the evaluatee.

The information on bacteria of the genus Corynebacterium present in the scalp flora of the evaluatee may include an abundance ratio of bacteria of the genus Corynebacterium in the scalp flora of the evaluatee, and the information on bacteria of the genus Corynebacterium present in the scalp flora of the evaluatee may consist of merely the abundance ratio of bacteria of the genus Corynebacterium in the scalp flora of the evaluatee. As the abundance ratio of bacteria of the genus Corynebacterium in the scalp flora is larger, at least any one of the following evaluations can be performed that a degree of the redness or the like is higher, that the number of gray hairs is larger, that the number of hairs is smaller, and that the variation in hair thickness is larger. For example, when an abundance ratio of bacteria of the genus Corynebacterium in a scalp flora is checked in a plurality of evaluatees of the same age group, at least any one of the following evaluations can be performed that an evaluatee having a higher abundance ratio has a higher degree of the scalp redness or the like, has a larger number of gray hairs, has a smaller number of hairs and has larger variation in hair thickness.

Besides, the evaluation of at least any one of the scalp redness or the like, the largeness in number of gray hairs, the smallness in number of hairs and the largeness of variation in hair thickness of the evaluatee can be performed by comparing the abundance ratio of bacteria of the genus Corynebacterium in the scalp flora of the evaluatee with a comparison criterion.

A prescribed value used as the comparison criterion is not especially limited, and can be, for example, any value of 11.0% or more and 14.0% or less, or any value of 12.0% or more and 13.0% or less. When the abundance ratio of bacteria of the genus Corynebacterium in the scalp flora of the evaluatee is smaller than the comparison criterion, it can be evaluated, regardless of the age, that there is a tendency of at least any one of a low degree of scalp redness or the like, a large number of hairs, and small variation in hair thickness.

The comparison criterion is preferably a value according to the age of the evaluatee. It is presumed that the abundance ratio of bacteria of the genus Corynebacterium in the scalp flora may vary according to the age, and when a comparison criterion according to the age of the evaluatee is used for the evaluation, the condition of the scalp and/or hair of the evaluatee can be more accurately evaluated.

Specifically, for example, information, of prescribed age groups each by 10 years or 5 years, on the abundance ratio of bacteria of the genus Corynebacterium in the scalp flora is precedently prepared, and the abundance ratio of bacteria of the genus Corynebacterium in the scalp flora actually measured in the evaluatee may be compared with the information of an age group to which the evaluatee belongs, so as to find the positioning of the abundance ratio of bacteria of the genus Corynebacterium of the evaluatee in the same age group. Here, the information of the prescribed age group is not especially limited, and may be, for example, an average or a medium of the abundance ratios of bacteria of the genus Corynebacterium of each age group, or an average or a deviation in each of plural ranks.

Besides, information on at least any one of the scalp redness or the like, the largeness in number of gray hairs, the smallness in number of hairs and the largeness of variation in hair thickness may be precedently prepared correspondingly to the information of each age group precedently prepared with regard to the abundance ratio of bacteria of the genus Corynebacterium in the scalp flora. In this manner, positioning, in the same age group, of the scalp redness or the like, the largeness in number of gray hairs, the smallness in number of hairs and the largeness of variation in hair thickness of the evaluatee can be evaluated at the same time as the evaluation of the positioning, in the age group of the evaluatee, of the abundance ratio of bacteria of the genus Corynebacterium.

### (1-2) Evaluation Method of Embodiment 2

An evaluation method of Embodiment 2 is a method for evaluating information on bacteria of the genus Corynebacterium present in a scalp flora of an evaluatee based on at least any one of scalp redness, an index related to the redness, a largeness in number of gray hairs, a smallness in number of hairs and a largeness of variation in hair thickness of the evaluatee.

The information on bacteria of the genus Corynebacterium present in the scalp flora of the evaluatee may include an abundance ratio of bacteria of the genus Corynebacterium in the scalp flora of the evaluatee, and the information on bacteria of the genus Corynebacterium present in the scalp flora of the evaluatee may consist of merely the abundance ratio of bacteria of the genus Corynebacterium in the scalp flora of the evaluatee. As a degree of the scalp redness or the like of the evaluatee is higher, the abundance ratio of bacteria of the genus Corynebacterium in the scalp flora of the evaluatee can be evaluated to be higher, as the number of gray hairs is larger, the abundance ratio of bacteria of the genus Corynebacterium in the scalp flora of the evaluatee can be evaluated to be higher, as the number of hairs is smaller, the abundance ratio of bacteria of the genus Corynebacterium in the scalp flora of the evaluatee can be evaluated to be higher, and as the variation in hair thickness is larger, the abundance ratio of bacteria of the genus Corynebacterium in the scalp flora of the evaluatee can be evaluated to be higher. For example, when at least any one of the scalp redness or the like, the largeness in number of gray hairs, the smallness in number of hairs and the largeness of variation in hair thickness is checked in a plurality of evaluatees of the same age group, an evaluatee having a higher degree of scalp redness can be evaluated to have a higher abundance ratio of bacteria of the genus Corynebacterium in the scalp flora, an evaluatee having a larger number of gray hairs can be evaluated to have a higher abundance ratio of bacteria of the genus Corynebacterium in the scalp flora, an evaluatee having a smaller number of hairs can be evaluated to have a higher abundance ratio of bacteria of the genus Corynebacterium in the scalp flora, and an evaluatee having larger variation in hair thickness can be evaluated to have a higher abundance ratio of bacteria of the genus Corynebacterium in the scalp flora.

Besides, when a reference comparison criterion for at least any one of the scalp redness or the like, the largeness in number of gray hairs, the smallness in number of hairs and the largeness of variation in hair thickness of an evaluatee is precedently grasped in association with a comparison criterion for the abundance ratio of bacteria of the genus Corynebacterium in the scalp flora corresponding to this reference comparison criterion, it can be evaluated, without measuring the abundance ratio of bacteria of the genus Corynebacterium but based on a comparison result with the reference comparison criterion for the scalp redness or the like, the largeness in number of gray hairs, the smallness in number of hairs or the largeness of variation in hair thickness of the evaluatee, whether or not the abundance ratio of bacteria of the genus Corynebacterium in the scalp flora of the evaluatee is higher than the comparison criteria.

A prescribed value used as the reference comparison criterion is not especially limited, and for the scalp redness or the like, an a* value, for example, can be any value of 8.0% or more and 16.0% or less, or any value of 9.0% or more and 12.0% or less, for the smallness in number of hairs, a total number of hairs in a unit area, for example, can be any value of 51 or more and 57 or less, or any value of 53 or more and 56 or less, and for the largeness of variation in hair thickness, a standard deviation of hair diameters (µm), for example, can be any value of 2.2 or more and 2.5 or less, or any value of 2.3 or more and 2.4 or less. When the scalp redness or the like of the evaluatee is lower than the reference comparison criterion, when the total number of hairs in a unit area of the evaluatee is larger than the reference comparison criterion, and when the standard deviation of hair diameters (µm) of the evaluatee is smaller than the reference comparison criterion, it can be evaluated, regardless of the age, that the abundance ratio of bacteria of the genus Corynebacterium in the scalp flora tends to be low.

It is noted that the reference comparison criterion is preferably a value according to the age of the evaluatee. In particular, the reference comparison criterion for the largeness in number of gray hairs (gray hair ratio) is preferably a value according to the age of the evaluatee.

Specifically, for example, information, of prescribed age groups each by 10 years or 5 years, on at least any one of the scalp redness or the like, the largeness in number of gray hairs, the smallness in number of hairs and the largeness of variation in hair thickness is precedently prepared, and any one of the scalp redness or the like, the largeness in number of gray hairs, the smallness in number of hairs and the largeness of variation in hair thickness found in an evaluatee may be compared with the corresponding information of an age group to which the evaluatee belongs, so as to find the positioning, in the same age group, of any one of the scalp redness or the like, the largeness in number of gray hairs, the smallness in number of hairs and the largeness of variation in hair thickness of the evaluatee. Here, the information of the prescribed age group is not especially limited, and may be, for example, an average or a medium of the scalp redness or the like, the largeness in number of gray hairs, the smallness in number of hairs and the largeness of variation in hair thickness of each age group, or an average or a deviation in each of plural ranks.

Besides, information on the abundance ratio of bacteria of the genus Corynebacterium in the scalp flora may be precedently prepared correspondingly to the information, of each age group, on at least any one of the scalp redness or the like, the largeness in number of gray hairs, the smallness in number of hairs and the largeness of variation in hair thickness. In this manner, the scalp redness or the like, the largeness in number of gray hairs, the smallness in number of hairs and the largeness of variation in hair thickness of the evaluatee in the same age group can be more accurately evaluated, and the abundance ratio of bacteria of the genus Corynebacterium of the same age group can be more accurately evaluated.

According to the evaluation methods of Embodiment 1 and Embodiment 2 described above, an evaluation method for a scalp and/or hair can be provided. It is noted that these evaluation methods may be employed for beauty purposes.

### (1-3) Evaluation Method of Embodiment 3

In an evaluation method according to Embodiment 3, at least any one of a largeness in number of gray hairs, a smallness in number of hairs, a largeness of variation in hair thickness and a thin hair ratio is evaluated according to scalp redness or an index related to the redness of an evaluatee.

### (1-4) Terms

Terms used in the description of the evaluation methods of Embodiment 1, Embodiment 2 and Embodiment 3 will now be specifically described as follows:
A scalp flora means an aggregation of a group of bacteria present in a scalp. The scalp flora can be analyzed, for example, by using a sample collected, with appropriate means such as an adhesive tape, from a partial area of a scalp corresponding to an evaluation target portion by a 16S rRNA sequencing method using a next generation DNA sequencer.

Bacteria of the genus Corynebacterium present in a scalp flora mean bacteria belonging to the genus Corynebacterium present in the scalp flora. Bacteria of the genus Corynebacterium present in the scalp flora can be analyzed, for example, by analyzing aresult of the scalp flora obtained by the 16S rRNA sequencing method described above at the level of the genus of the bacteria with an analysis tool for pipeline.

An abundance ratio of bacteria of the genus Corynebacterium in a scalp flora means a ratio [(C)/(T)] of the bacteria (C) of the genus Corynebacterium to whole bacteria (T) present in the scalp flora obtained based on the analysis result of the scalp flora. More specifically, it refers to a ratio [(C)/(T)] obtained through analysis of the result of the scalp flora obtained by the 16S rRNA sequencing method at the level of the genus of the bacteria with an analysis tool for pipeline.

Redness is not especially limited, and may be a degree of redness felt through visual observation or observation using imaging means such as a microscope, and in particular, from the viewpoint of improving objectivity of the evaluation, it preferably means a chromaticity a* value in L*a*b* color space found from an image of an evaluation target portion of a scalp obtained by the imaging means such as a microscope. For example, when the a* value of a scalp of an evaluatee is higher than the a* value of a scalp of a person corresponding to a prescribed reference, it is determined that the scalp of the evaluatee has a higher degree of redness than the scalp of the person corresponding to the prescribed reference.

An index related to the redness is not especially limited, and may be a degree of feeling a color of blue, green, blue green or the like in a substantially complementary relationship with red through visual observation or observation with imaging means such as a microscope, and from the viewpoint of improving objectivity of the evaluation, it preferably means a chromaticity a* value in the L*a*b* color space found from an image of an evaluation target portion of a scalp obtained by the imaging means such as a microscope. For example, when the a* value of a scalp of an evaluatee is lower than the a* value of a scalp of a person corresponding to a prescribed reference, it is determined that the scalp of the evaluatee has a lower degree of redness than the scalp of the person corresponding to the prescribed reference.

As an index related to the largeness in number of gray hairs, for example, a ratio of gray hairs in all hairs growing in a prescribed unit area in an evaluation target portion of a scalp (a gray hair ratio) can be used. The prescribed unit area is not especially limited, and may be, for example, a range displayed in a screen when imaging means such as a microscope is used at a prescribed magnification (the same shall apply hereinafter).

As an index related to the smallness in number of hairs, a total number of hairs growing in a prescribed unit area of an evaluation target portion of a scalp may be used, or a ratio of black region obtained by subjecting, to black-and-white binarization, an image obtained by imaging means such as a microscope may be used.

As an index related to the largeness of variation in hair thickness, for example, a standard deviation of hair diameters of hairs growing in a prescribed unit area in an evaluation target portion of a scalp may be used. It is noted that a hair diameter can be measured by appropriate means.

When a thin hair ratio is to be evaluated, it is evaluated that the thin hair ratio tends to be higher as a degree of the scalp redness of an evaluatee is higher, and that the thin hair ratio tends to be lower as the degree of the scalp redness of the evaluatee is lower. Here, in the evaluation of the thin hair ratio, a ratio of number of thin hairs (thin hair ratio) having a hair diameter of 40 µm or less in a total number of hairs growing in a prescribed unit area in an evaluation target portion of a scalp can be used.

For this evaluation, from the viewpoint of finding a current objective condition, information corresponding to a comparison criterion for the scalp redness or the like according to the age of the evaluatee is preferably used.

Preferably, for example, information, of prescribed age groups each by 10 years or 5 years, on the scalp redness or the like of a plurality of persons (data in numerical form corresponding to redness or the like, or image data of a scalp) is precedently prepared, and information on the scalp redness of the age group to which the age of the evaluatee belongs is used as a comparison criterion to be compared with the scalp redness or the like of the evaluatee for the evaluation.

Although a proportion of persons having scalp redness and the degree of the redness are varied according to the age, the positioning, in persons of the same age, of the condition of the scalp of an evaluatee can be objectively found through comparison with information of the same age group. Besides, a plurality of stages (of, for example, 3 stages or 5 stages) divided according to the degree of the redness or the like of each age group may be precedently examined and prepared, so that the evaluation can be performed by specifying a stage corresponding to the scalp redness or the like of the evaluatee. Alternatively, a deviation related to the degree of the redness or the like of each age group may be precedently examined and prepared, so that the evaluation can be performed by specifying a deviation corresponding to the scalp redness or the like of the evaluatee.

Besides, when the information on the scalp redness or the like of a plurality of age groups is used, a risk of at least any one of increasing the number of gray hairs, reducing the number of hairs, increasing the variation in hair thickness and the thin hair ratio can be predicted assuming that the evaluatee gets older with the current scalp redness or the like kept, or assuming that the current degree of the scalp redness or the like is changed through aging of the evaluatee.

Specifically, when the degree of the scalp redness or the like of the evaluatee is evaluated to be high in the same age group (for example, to be within top 30% regarding the redness), it can be predicted that there is a risk that at least any one of increasing the number of gray hairs, reducing the number of hairs, increasing the variation in hair thickness and the thin hair ratio may become equivalent to that of a person having a high degree of the scalp redness of an older age group (for example, similarly to be within top 30%) if the evaluatee gets older with the current scalp redness kept.

Alternatively, when the degree of the scalp redness or the like of the evaluatee is evaluated to be low in the same age group (for example, to be within bottom 30% regarding the redness), it can be predicted that there is a tendency that at least any one of the largeness in number of gray hairs, the smallness in number of hairs, the largeness of variation in hair thickness and the thin hair ratio can be kept equivalently to that of a person having a low degree of the scalp redness of an older age group (for example, similarly to be within bottom 30%) if the evaluatee can keep the scalp redness at the current low degree even through aging.

Alternatively, when the degree of the scalp redness or the like of the evaluatee is evaluated to be high in the same age group (for example, to be within top 30% regarding the redness), it can be predicted that at least any one of the largeness in number of gray hairs, the smallness in number of hairs, the largeness of variation in hair thickness and the thin hair ratio may become close to that of a person having a low degree of the scalp redness of an older age group (for example, similarly to be within bottom 30%) if the evaluatee gets older with the current degree of the scalp redness lowered.

In particular, with respect to the largeness in number of gray hairs, a person evaluated to have a high degree of the scalp redness or the like in the same age group tends to have gray hairs rapidly increasing when he/she becomes older than about 40 years. Therefore, it can be predicted that an evaluatee younger than 40 years and having a high degree of the scalp redness has a risk of rapid increase in number of gray hairs in the future if he/she gets older with the current scalp redness kept.

Besides, when the evaluation is performed by using a plurality of information of respective age groups, an age group having, as an average, redness or the like corresponding to the current scalp redness or the like of the evaluatee can be presumed, namely, a skin age of the scalp of the evaluatee can be presumed.

As the evaluation target portion of the scalp, for example, various portions such as a top portion (top of a head), a front portion (frontal region), a side portion (above an ear), a back portion (occipital region) and a nape portion (below the occipital region, neckline) can be used, and a top portion (top of a head) and/or a front portion (frontal region) that can be easily observed visually or with a microscope is preferred, and from the viewpoint of suppressing the influence on the evaluation of sunburn of the scalp, a top portion (top of a head) excluding a parting or whorl of hair is preferably used as the evaluation target portion. Here, a top portion (top of a head) refers to a portion of the scalp within a radius of 3 cm from the highest position of the top of the head. From the viewpoint of improving objectivity of the evaluation, the same evaluation target portions are preferably used for the comparison.

### (2) Selection Method

A selection method is a method for selecting a scalp/hair cosmetic to be recommended to an evaluatee according to the evaluation result obtained by the evaluation method of Embodiment 1 or the evaluation method of Embodiment 2 described above. Besides, the selection method is a method for selecting a hair treatment agent to be recommended to an evaluatee according to the evaluation result obtained by the evaluation method of Embodiment 3 described above.

For example, when an evaluatee is evaluated, by the evaluation method of Embodiment 1 or the evaluation method of Embodiment 2, to have a high abundance ratio of bacteria of the genus Corynebacterium in the scalp flora, since he/she tends to have a high degree of scalp redness, a scalp/hair cosmetic containing a component capable of lowering the degree of scalp redness can be selected and recommended to use it, or he/she is recommended not to use a scalp/hair cosmetic containing a component possibly increasing the degree of scalp redness. Besides, for example, an evaluatee evaluated to have a high degree of scalp redness by the evaluation method of Embodiment 3 is recommended to use a hair treatment agent containing a component capable of lowering the degree of scalp redness, or not to use a hair treatment agent containing a component possibly increasing the degree of scalp redness.

The term "scalp/hair cosmetic" as used herein means a cosmetic used for a scalp and/or hair.

For the evaluation method of Embodiment 1 or the evaluation method of Embodiment 2, scalp/hair cosmetics to be recommended to use or scalp/hair cosmetics to be recommended not to use according to the degree of an abundance ratio of bacteria of the genus Corynebacterium in the scalp flora may be precedently distinguishably listed in a table, so that a scalp/hair cosmetic to be recommended can be selected by using the table. Besides, for the evaluation method of Embodiment 3, hair treatment agents to be recommended to use or hair treatment agents to be recommended not to use according to the degree of scalp redness or the like may be precedently listed in a table distinguishably according to the degree of scalp redness or the like, so that a hair treatment agent to be recommended can be selected by using the table.

Besides, an evaluatee evaluated to have a high degree of scalp redness or the like, a large number of gray hairs, a small number of hairs, or large variation in hair thickness tends to have a high abundance ratio of bacteria of the genus Corynebacterium in the scalp flora and a high degree of scalp redness or the like, and therefore, a scalp/hair cosmetic or the like can be selected in the same manner as described above.

Besides, based on the evaluation result obtained by the evaluation method of Embodiment 1 or the evaluation method of Embodiment 2 described above, a scalp/hair cosmetic containing an anti-inflammatory agent is preferably selected.

More specifically, an evaluatee evaluated to have a high abundance ratio of bacteria of the genus Corynebacterium in the scalp flora by the evaluation method of Embodiment 1 or the evaluation method of Embodiment 2 tends to have a high degree of scalp redness or the like, and hence, use of a scalp/hair cosmetic containing an anti-inflammatory agent is recommended. An evaluatee evaluated to have a low abundance ratio of bacteria of the genus Corynebacterium in the scalp flora tends to have a low degree of scalp redness or the like, and hence, use of a scalp/hair cosmetic not limited to a scalp/hair cosmetic containing an anti-inflammatory agent (for example, use of a scalp/hair cosmetic taking precedence on improvement of hair condition over improvement of scalp condition) can be recommended.

Besides, based on the evaluation result obtained by the evaluation method of Embodiment 3 described above, a hair treatment agent containing an anti-inflammatory agent is preferably selected.

More specifically, to an evaluatee evaluated to have a high degree of scalp redness or the like by the evaluation method of Embodiment 3, use of a hair treatment agent containing an anti-inflammatory agent is recommended, and to an evaluate evaluated to have a low degree of scalp redness or the like, use of a hair treatment agent not limited to a hair treatment agent containing an anti-inflammatory agent (for example, use of a hair treatment agent taking precedence on improvement of hair condition over improvement of scalp condition) can be recommended.

As the anti-inflammatory agent, one, two or more of β-glycyrrhetinic acid, dipotassium glycyrrhizinate, trisodium glycyrrhizinate, monoammonium glycyrrhizinate, pyridoxine hydrochloride, nicotinic acid, nicotinamide and allantoin can be used.

A content of the anti-inflammatory agent in a scalp/hair cosmetic is, from the viewpoint that scalp redness can be reduced, preferably 0.05% by mass or more and 5% by mass or less, and more preferably 0.1% by mass or more and 2% by mass or less. For an evaluatee evaluated to have a higher abundance ratio of bacteria of the genus Corynebacterium in the scalp flora or evaluated to have a higher degree of scalp redness according to the evaluation result, the content of the anti-inflammatory agent in a scalp/hair cosmetic may be determined to be higher.

Examples of the scalp/hair cosmetic include a shampoo, a rinse, a conditioner, a treatment (such as a leave-in treatment, a rinse off treatment, a styling treatment, a constituent agent of a multiconstituent treatment, a treatment for use in pretreatment for perm, a treatment for use in post treatment for perm, a treatment for use in pretreatment for hair dyeing, a treatment for use in post treatment for hair dyeing, a treatment for use in pretreatment for hair bleaching and a treatment for use in post treatment for hair bleaching), a hair dressing agent and a hair growing agent.

Such a scalp/hair cosmetic may be in a dosage form of, for example, a liquid, a cream, a gel, a foam or a spray.

The scalp/hair cosmetic can contain substances known as raw materials in a scalp/hair cosmetic, including a cationic surfactant, an amphoteric surfactant, a nonionic surfactant, a higher alcohol, a polyhydric alcohol, a lower alcohol, a saccharide, an ester oil, fats and oils, fatty acid, hydrocarbon, a wax, silicone, a polymer compound, an amino acid, an animal/plant extract, a microbial substance, an inorganic compound, a perfume, a preservative, a sequestering agent, and a UV absorber.

### (3) Evaluation Support Tool

An evaluation support tool is an evaluation support tool for supporting evaluation in the evaluation method of Embodiment 1 or evaluation in the evaluation method of Embodiment 2 described above, and may display a comparison criterion for an abundance ratio of bacteria of the genus Corynebacterium in a scalp flora. Besides, the evaluation support tool is an evaluation support tool for supporting evaluation in the evaluation method of Embodiment 3, namely, an evaluation support tool for supporting evaluation of at least any one of a largeness in number of gray hairs, a smallness in number of hairs, a largeness of variation in hair thickness and a thin hair ratio, and may display information corresponding to a comparison criterion to be compared with scalp redness or the like of an evaluatee.

The evaluation support tool is not especially limited, and the evaluation support tool for supporting the evaluation in the evaluation method of Embodiment 1 or the evaluation in the evaluation method of Embodiment 2 can be, for example, a sheet explaining the condition for a scalp and/or hair in which information corresponding to a comparison criterion of an abundance ratio of bacteria of the genus Corynebacterium in the scalp flora is described, a pamphlet in which types of scalp/hair cosmetics to be recommended according to an abundance ratio of bacteria of the genus Corynebacterium in a scalp flora are described, an information terminal capable of displaying, in a screen, information corresponding to a comparison criterion for an abundance ratio of bacteria of the genus Corynebacterium in a scalp flora and various data and programs to be used in the information terminal, or an information terminal capable of displaying, in a screen, types of scalp/hair cosmetics to be recommended according to an abundance ratio of bacteria of the genus Corynebacterium in a scalp flora and various data and programs to be used in the information terminal.

Furthermore, the evaluation support tool for supporting the evaluation in the evaluation method of Embodiment 1 or the evaluation in the evaluation method of Embodiment 2 can be, for example, a sheet explaining the condition of a scalp and/or hair in which an abundance ratio of bacteria of the genus Corynebacterium in a scalp flora is described in association with at least any one of scalp redness, an index related to the redness, the largeness in number of gray hairs, the smallness in number of hairs and the largeness of variation in hair thickness, a pamphlet in which types of corresponding scalp/hair cosmetics are further described, an information terminal storing, in a displayable manner, information on an abundance ratio of bacteria of the genus Corynebacterium in a scalp flora in association with at least any one of scalp redness, an index related to the redness, the largeness in number of gray hairs, the smallness in number of hairs and the largeness of variation in hair thickness and various data and programs to be used in the information terminal, or an information terminal further storing, in a displayable manner, information on types of corresponding scalp/hair cosmetics and various data and programs to be used in the information terminal.

The information terminal working as the evaluation support tool for supporting the evaluation in the evaluation method of Embodiment 1 or the evaluation in the evaluation method of Embodiment 2 is not especially limited, and can be, for example, an information terminal that includes a display for displaying a correspondence table or the like showing correspondence between an abundance ratio of bacteria of the genus Corynebacterium in a scalp flora and at least any one of scalp redness, an index related to the redness, the largeness in number of gray hairs, the smallness in number of hairs and the largeness of variation in hair thickness, a CPU for performing various information processing, a RAM, recording means storing various data and programs used in the evaluation of an evaluatee, such as a ROM, and input means for receiving information input from a user, such as a touch panel or a keyboard, and is configured to be able to perform communication via network such as the Internet. Here, the various data used in the evaluation of an evaluatee may be precedently stored in the recording means of the evaluation support tool, or the evaluation support tool may be configured to acquire the data from an external server or the like through the communication via network to be stored therein. For example, when information of the respective age groups is stored in the recording means, the information terminal may cause the CPU to read the information of a corresponding age group based on age information of an evaluatee received through the input means to be output to and displayed in the display.

Besides, the evaluation support tool for supporting the evaluation in the evaluation method of Embodiment 3 can be, for example, a sheet explaining the condition of a scalp in which information corresponding to a comparison criterion to be used for evaluating scalp redness or the like is described, a pamphlet in which a hair treatment agent to be recommended according to the condition of scalp redness or the like is described in association with the redness or the like, an information terminal capable of displaying, in a screen, information corresponding to a comparison criterion for evaluating the scalp redness or the like and various data and programs to be used in the information terminal, or an information terminal capable of displaying, in a screen, a hair treatment agent to be recommended according to the condition of the scalp redness or the like and various data and programs to be used in the information terminal.

The information terminal working as the evaluation support tool for supporting the evaluation in the evaluation method of Embodiment 3 is not especially limited, and can be, for example, an information terminal that includes a display for displaying image data and the like of a scalp, a CPU for performing various information processing, a RAM, recording means storing various data and programs used in the evaluation of an evaluatee, such as a ROM, and input means for receiving information input from a user, such as a touch panel or a keyboard, and is configured to be able to perform communication via network such as the Internet. Here, information used in the evaluation of an evaluatee may be precedently stored in the recording means, or may be acquired from an external server or the like through the communication via network to be stored. In using such an information terminal, for example, when information of an age group and the like of the evaluatee input through the input means is accepted, the CPU reads information of the corresponding age group from the recording means to be output to and displayed in the display, and thus, information corresponding to a comparison criterion to be compared with the scalp redness or the like of the evaluatee can be found.

The information displayed in the evaluation support tool for supporting the evaluation in the evaluation method of Embodiment 3 and corresponding to a comparison criterion to be compared with the scalp redness or the like of the evaluatee is preferably information corresponding to a comparison criterion for the scalp redness or the like according to the age of the evaluatee from the viewpoint of finding a current objective condition of the evaluatee.

In particular, from the viewpoints that the positioning, in the same age group, of the scalp condition of an evaluatee can be objectively found, and that a risk of at least any one of increasing the number of gray hairs, reducing the number of hairs, increasing the variation in hair thickness and a thin hair ratio caused when the evaluatee gets older with the current scalp redness or the like kept can be predicted, the evaluation support tool preferably displays data in numerical form of the redness or the like or image data of the scalp with respect to each of the plural age groups and with respect to each degree of the scalp redness or the like, and such data is preferably displayed in the form of a table.

### (4) Screening Method for Scalp/Hair Cosmetic

A screening method for a scalp/hair cosmetic is not especially limited, and may be, for example, a method including the steps of: treating a scalp and/or hair of an evaluatee with a scalp/hair cosmetic at least once; and comparing abundance ratios of bacteria of the genus Corynebacterium in a scalp flora of the evaluatee before and after the step of treating a scalp and/or hair. Here, the number of times of treating a scalp and/or hair is not limited to once, but can be, for example, a prescribed number of times such as 30 times or 90 times.

When the abundance ratio of bacteria of the genus Corynebacterium in the scalp flora of the evaluatee can be lowered by treating the scalp and/or hair with a prepared scalp/hair cosmetic, this scalp/hair cosmetic can be selected as an advantageous one for attaining effects of suppressing the scalp redness or the like, reducing the number of gray hairs, suppressing the decrease in number of hairs, and suppressing the variation in hair thickness.

One that can more largely lower the abundance ratio of bacteria of the genus Corynebacterium in the scalp flora may be selected as one capable of more sufficiently attaining the effects. Also examination of a degree of lowering the abundance ratio of bacteria of the genus Corynebacterium here is performed preferably based on the abundance ratio of an age group of the evaluatee for whom the screening method for a scalp/hair cosmetic is performed.

Alternatively, the screening method for a scalp/hair cosmetic includes, for example, the steps of: treating a scalp and/or hair of an evaluatee with a scalp/hair cosmetic at least once; and comparing scalp redness or the like of the evaluatee before and after the step of treating a scalp and/or hair. Here, the number of times of treating a scalp and/or hair is not limited to once, but can be, for example, a prescribed number of times such as 30 times or 90 times.

When the scalp redness or the like of the evaluatee can be reduced by treating the scalp and/or hair with a prepared scalp/hair cosmetic, this scalp/hair cosmetic can be selected as an advantageous one for attaining effects of reducing the number of gray hairs, suppressing the decrease in number of hairs, suppressing the variation in hair thickness, and suppressing the thin hair ratio.

One that can more largely reduce the scalp redness or the like may be selected as one capable of more sufficiently attaining the effects. Also examination of a degree of reducing the scalp redness or the like here is performed preferably based on the abundance ratio of an age group of the evaluatee for whom the screening method for a scalp/hair cosmetic is performed.

### (5) Screening Method for Candidate Substance for Active Ingredient

A screening method for a candidate substance for an active ingredient is not especially limited, and for example, includes the steps of: treating a scalp and/or hair of an evaluatee with a candidate substance for an active ingredient at least once; and comparing abundance ratios of bacteria of the genus Corynebacterium in a scalp flora of the evaluatee before and after the step of treating a scalp and/or hair. Here, the number of times of treating a scalp and/or hair is not limited to once, but can be, for example, a prescribed number of times such as 30 times or 90 times.

Here, a candidate substance for an active ingredient may be a known component that can be contained in a scalp/hair cosmetic. It is noted that a method for treating a scalp and/or hair may be an appropriate method that can be usually performed.

When the abundance ratio of bacteria of the genus Corynebacterium in the scalp flora of the evaluatee can be lowered by treating the scalp and/or hair with a candidate substance for an active ingredient, this candidate substance can be selected as an advantageous active ingredient for attaining effects of suppressing the scalp redness or the like, reducing the number of gray hairs, suppressing the decrease in number of hairs, and suppressing the variation in hair thickness.

One that can more largely lower the abundance ratio of bacteria of the genus Corynebacterium in the scalp flora may be selected as one capable of more sufficiently attaining the effects. Also examination of a degree of lowering the abundance ratio of bacteria of the genus Corynebacterium here is performed preferably based on the abundance ratio of an age group of the evaluatee for whom the screening method for a candidate substance for an active ingredient is performed.

Alternatively, the screening method for a candidate substance for an active ingredient includes, for example, the steps of: treating a scalp and/or hair of an evaluatee with a candidate substance for an active ingredient at least once; and comparing scalp redness or the like of the evaluatee before and after the step of treating a scalp and/or hair. Here, the number of times of treating a scalp and/or hair is not limited to once, but can be, for example, a prescribed number of times such as 30 times or 90 times.

Here, a candidate substance for an active ingredient may be a known component that can be contained in a scalp/hair cosmetic. It is noted that a method for treating a scalp and/or hair may be an appropriate method that can be usually performed.

When the scalp redness or the like of the evaluatee can be reduced by treating the scalp and/or hair with a candidate substance for an active ingredient, this candidate substance can be selected as an advantageous active ingredient for attaining effects of reducing the number of gray hairs, suppressing the decrease in number of hairs, suppressing the variation in hair thickness, and suppressing the thin hair ratio.

One that can more largely reduce the scalp redness or the like may be selected as one capable of more sufficiently attaining the effects. Also examination of a degree of reducing the scalp redness or the like here is performed preferably based on the abundance ratio of an age group of the evaluatee for whom the screening method for a candidate substance of an active ingredient is performed.

### (6) Screening Method for Selecting Evaluatee

A screening method for selecting an evaluatee is not especially limited, and may include, for example, the steps of: checking an abundance ratio of bacteria of the genus Corynebacterium in a scalp flora of an evaluatee; and comparing the abundance ratio of the evaluatee with a comparison criterion for an abundance ratio of bacteria of the genus Corynebacterium in a scalp flora.

When the abundance ratio of bacteria of the genus Corynebacterium in the scalp flora of the evaluatee thus checked was higher than the comparison criterion, the evaluatee can be selected as an evaluatee having at least any one of a tendency to a higher degree of scalp redness or the like, a tendency to a larger number of gray hairs, a tendency to a smaller number of hairs and a tendency to large variation in hair thickness as compared with a person corresponding to the comparison criterion. Alternatively, when the abundance ratio of bacteria of the genus Corynebacterium in the scalp flora of the evaluatee thus checked was lower than the comparison criterion, the evaluatee can be selected as an evaluatee having at least any one of a tendency to a lower degree of scalp redness or the like, a tendency to a smaller number of gray hairs, a tendency to a larger number of hairs and a tendency to small variation in hair thickness as compared with the person corresponding to the comparison criterion. Thus, counseling can be performed, for example, to propose a scalp/hair cosmetic according with the selected evaluatee.

In the selection of an evaluatee, the selection may be performed according to a degree of divergence, from the comparison criterion, of the checked abundance ratio of bacteria of the genus Corynebacterium in the scalp flora of the evaluatee.

Besides, also in the step of comparing, in the abundance ratio of bacteria of the genus Corynebacterium in the scalp flora, the measured value of the evaluatee with the comparison criterion, the comparison criterion for the abundance ratio of an age group of the evaluatee is preferably used for the comparison.

Alternatively, the screening method for selecting an evaluatee may include, for example, the steps of: checking a degree of scalp redness or the like of an evaluatee; and comparing the scalp redness or the like of the evaluatee with a comparison criterion for the scalp redness or the like.

When the checked scalp redness or the like of the evaluatee is at a higher degree than the comparison criterion, the evaluatee can be selected as an evaluatee having at least any one of a tendency to a larger number of gray hairs, a tendency to a smaller number of hairs, a tendency to large variation in hair thickness and a tendency to a higher thin hair ratio as compared with a person corresponding to the comparison criterion. Alternatively, when the checked scalp redness or the like of the evaluatee is at a lower degree than the comparison criterion, the evaluatee can be selected as an evaluatee having at least any one of a tendency to a lower degree of scalp redness, a tendency to a smaller number of gray hairs, a tendency to a larger number of hairs, a tendency to smaller variation in hair thickness and a tendency to a lower thin hair ratio as compared with a person corresponding to the comparison criterion. Thus, counseling can be performed, for example, to propose a scalp/hair cosmetic according with the selected evaluatee.

In the selection of an evaluatee, the selection may be performed according to a degree of divergence, from the comparison criterion, of the checked scalp redness or the like of the evaluatee.

Besides, also in the step of comparing, in the scalp redness or the like, the measured value of the evaluatee with the comparison criterion, the comparison criterion for the abundance ratio of an age group of the evaluatee is preferably used for the comparison.

### EXAMPLES

In sixty subjects (sixty Japanese women classified into Mongoloid) belonging to each of a group of 30s (30 to 39 years old) and a group of 50s (50 to 59 years old), an image of a scalp of a top portion (top of a head) was taken at a magnification of 40x using a microscope (DIGITAL MICROSCOPE KH-8700 manufactured by HiROX Co., Ltd.). Subsequently, a scalp flora was collected from the scalp of the top portion (top of a head) by using an adhesive tape. It is noted that the scalp whose image was taken and the scalp from which the scalp flora was collected were both a scalp within a radius of 3 cm from the highest position of the top of the head and including neither a parting nor whorl.

Image data of the scalps of the top portions (tops of the heads) obtained with the microscope and a* values found from the image data were used to divide the subjects of each of the group of 30s and the group of 50s into a group having a high degree of scalp redness (ten subjects having highest a* values) and a group having a low degree of scalp redness (ten subjects having lowest a* values). Besides, an average of the a* values of the ten subjects was obtained in each group.

A sample of each scalp flora collected from the group having a high degree of scalp redness and the group having a low degree of scalp redness was analyzed by the 16S rRNA sequencing method according to the following procedures [1] to [4]:
[1] A nucleic acid of the scalp flora collected from the adhesive tape was extracted by an ordinary method.
[2] The extracted nucleic acid was used for amplifying 16S rRNA gene by PCR.
[3] The amplified 16S rRNA gene was used for DNA analysis of the 16S rRNA gene using a next generation sequencer (Milseq (product name) manufactured by Illumina Inc.).
[4] Based on the thus obtained analysis result, abundance ratios at the level of genus of various bacteria in the collected scalp flora were obtained using an analysis tool, QIIME.

Besides, in each of the subjects in their 30s or 50s divided into the group having a high degree of scalp redness (ten subjects having the highest a* values) and the group having a low degree of scalp redness (ten subjects having the lowest a* values), a "gray hair ratio", a "total number of hairs in a unit area" and a "standard deviation of hair diameters (µm)" were obtained based on the microscope image of the top portion of the scalp (top of the head) obtained with the microscope.

Here, an average, in ten subjects of each group, of total numbers of hairs growing in each scalp in an image area shown in the microscope image of each subject was defined as the "total number of hairs in a unit area". Besides, an average, of ten subjects of each group, of ratios in number of gray hairs in the total number of hairs growing in the scalp in the image area shown in the microscope image of the subject was defined as the "gray hair ratio". Furthermore, a standard deviation of each group calculated based on diameters of ten hairs randomly selected from the hairs growing in the scalp in the image area shown in the microscope image of the subject was defined as the "standard deviation of hair diameters (µm)".

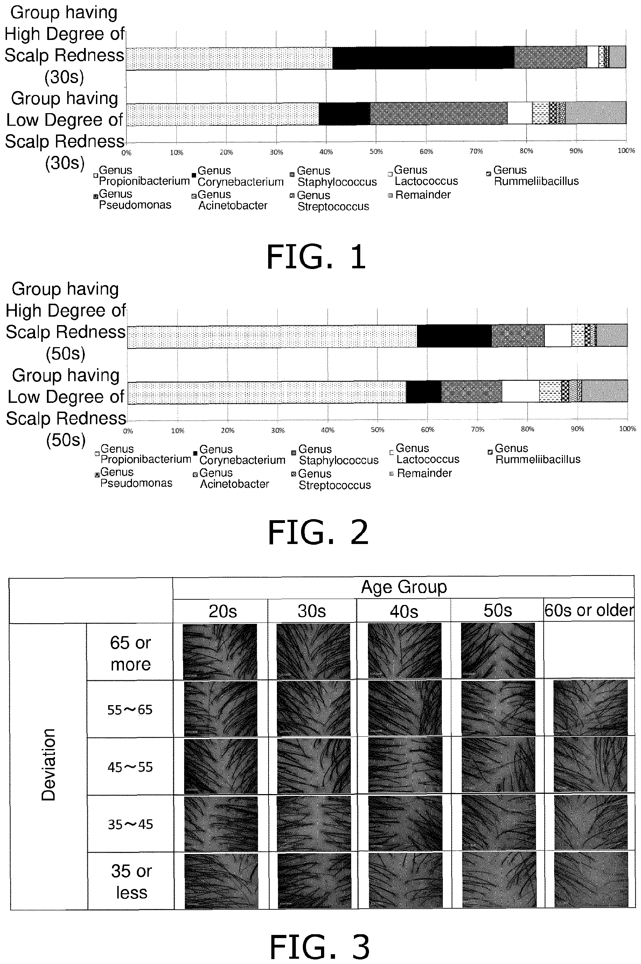

Table 1 shows, with respect to each of the group having a high degree of scalp redness (average of ten subjects) and the group having a low degree of scalp redness (average of ten subjects) of the subjects in their 30s and 50s, an average of the a* values of each group, the analysis result of the scalp flora (average of ten subjects), an average of the gray hair ratios, an average of the total numbers of hairs in a unit area, and a standard deviation of hair diameters (µm) of each group. Besides, the analysis results of Table 1 are shown in the form of graphs in Figures 1 and 2. Here, with respect to genera of bacteria whose abundance ratios in the scalp flora were 1% or less, a sum of their abundance ratios is shown as "Remainder" (Table 1 and Figures 1 and 2).

**[Table 1]**

| | | Age Group of 30s | | Age Group of 50s | |
|---|---|---|---|---|---|
| | | Group having Low Scalp Redness (10 subjects) | Group having High Scalp Redness (10 subjects) | Group having Low Scalp Redness (10 subjects) | Group having High Scalp Redness (10 subjects) |
| a* Value | | 7.69 | 17.1 | 5.84 | 18.72 |
| Scalp Flora | Genus Propionibacterium | 38.6% | 41.4% | 55.7% | 58.0% |
| | Genus Corynebacterium | 10.2% | 36.3% | 7.0% | 14.8% |
| | Genus Staphylococcus | 27.4% | 14.6% | 12.1% | 10.5% |
| | Genus Lactococcus | 5.1% | 2.4% | 7.6% | 5.6% |
| | Genus Rummeliibacillus | 3.4% | 1.0% | 4.4% | 2.6% |
| | Genus Pseudomonas | 1.4% | 0.4% | 1.4% | 1.1 % |
| | Genus Acinetobacter | 0.6% | 0.4% | 1.7% | 0.9% |
| | Genus Streptococcus | 1.2% | 0.1% | 0.9% | 0.3% |
| | Remainder | 12.1% | 3.4% | 9.2% | 6.2% |
| Gray Hair Ratio | | 1.4% | 2.2% | 9.2% | 50.3% |
| Total Number of Hairs in Unit Area | | 58 | 49.5 | 58.4 | 47.3 |
| Standard Deviation of Hair Diameters (µm) | | 2.1 | 3.1 | 2.1 | 2.6 |

It was revealed, based on the results shown in Table 1 and Figures 1 and 2, that the abundance ratio of bacteria of the genus Corynebacterium in the scalp flora was 36.3% (ratio [(C)/(T)] of bacteria of the genus Corynebacterium in the scalp flora to all the bacteria (T) in the scalp flora: 0.363) in the group having a high degree of scalp redness, and was 10.2% (ratio [(C)/(T)]: 0.102) in the group having a low degree of scalp redness of the subjects in their 30s. Besides, it was revealed that the abundance ratio of bacteria of the genus Corynebacterium in the scalp flora was 14.8% (ratio [(C)/(T)] of bacteria of the genus Corynebacterium in the scalp flora to all the bacteria (T) in the scalp flora: 0.148) in the group having a high degree of scalp redness, and was 7.0% (ratio [(C)/(T)]: 0.070) in the group having a low degree of scalp redness of the subjects in their 50s.

In all the groups of the subjects in their 30s and 50s, an abundance ratio in the scalp flora of the genus Propionibacterium was the highest, but the abundance ratio of the genus Propionibacterium was not varied depending on scalp redness or the like, the largeness in number of gray hairs, the smallness in number of hairs and the largeness of variation in hair thickness.

Besides, it is understood from the results shown in Table 1 and Figures 1 and 2 that, in all the subjects in their 30s and 50s, the abundance ratio of bacteria of the genus Corynebacterium in the scalp flora is high in the group having a high degree of scalp redness as compared with the group having a low degree of scalp redness, and that a difference therebetween is large (twice or more).

It was revealed from the results shown in Table 1 that, in all the subjects of their 30s and 50s, the "gray hair ratio" and the "standard deviation of hair diameters (µm)" were higher and the "total number of hairs in a unit area" was smaller in the group having a high degree of scalp redness than in the group having a low degree of scalp redness. These results reveal the following:
<1> It is understood that the number of gray hairs tends to be larger and the abundance ratio of bacteria of the genus Corynebacterium in the scalp flora tends to be higher as the degree of scalp redness is higher. Besides, there is a tendency that the abundance ratio of bacteria of the genus Corynebacterium in the scalp flora is higher as the number of gray hairs is larger.
<2> It is understood that the number of hairs tends to be smaller and the abundance ratio of bacteria of the genus Corynebacterium in the scalp flora tends to be higher as the degree of scalp redness is higher. Besides, there is a tendency that the abundance ratio of bacteria of the genus Corynebacterium in the scalp flora is higher as the number of hairs is smaller.
<3> It is understood that the variation in hair thickness tends to be larger and the abundance ratio of bacteria of the genus Corynebacterium in the scalp flora tends to be higher as the degree of scalp redness is higher. Besides, there is a tendency that the abundance ratio of bacteria of the genus Corynebacterium in the scalp flora is higher as the variation in hair thickness is larger.

Apart from the items shown in Table 1 and Figures 1 and 2, Figure 3 illustrates image data of scalps obtained, with a microscope, from 281 subjects (281 Japanese women classified into Mongoloid) belonging to a plurality of age groups shown according to the age groups (each by 10 years) and according to deviations of the a* values obtained from the image data.

Here, each deviation was obtained by using the a* values of each age group. For example, image data of scalps shown in a row corresponding to deviation values of 45 to 55 of subjects in their 40s corresponds to image data of scalps of subjects in their 40s and having a deviation of the a* value of 45 to 55.

Referring to Figure 3 described above, when a microscope image of the scalp of a subject is compared with each scalp image corresponding to a column (vertical column) of the age group of an evaluatee, a deviation in the corresponding age group of scalp image of the evaluatee can be specified, and positioning of the scalp redness of the evaluatee in the same age group can be evaluated.

Furthermore, in addition to a list of the scalp redness or the like illustrated in Figure 3, and correspondingly to the list, the following lists may be previously prepared: a list of abundance ratios of bacteria of the genus Corynebacterium in the scalp floras according to respective age groups and respective deviations; a list of the largeness in number of gray hairs according to the age groups and the deviations; a list of the smallness in number of hairs according to the age groups and the deviations; and a list of the largeness of variation in hair thickness according to the age groups and the deviations. In this manner, based on the deviation of the redness of the subject in the corresponding age group, positionings of the evaluatee, in the same age group, of the abundance ratio of bacteria of the genus Corynebacterium, the largeness in number of gray hairs, the smallness in number of hairs and the largeness of variation in hair thickness can be also evaluated.

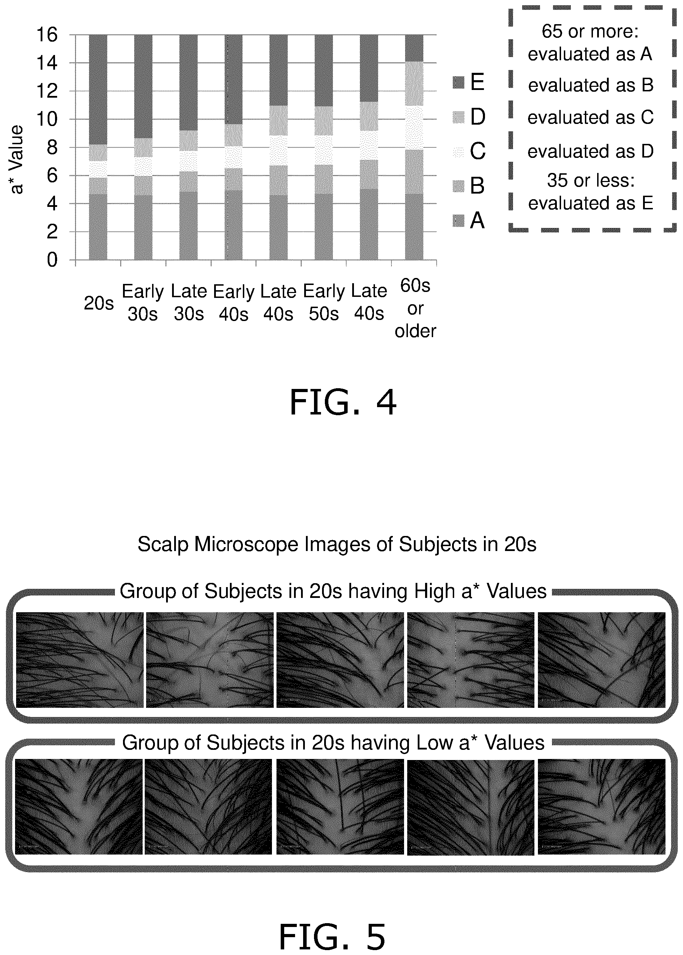

The list of the redness or the like is not limited to one illustrated in Figure 3, but may be, for example, one illustrated in Figure 4 in which deviations of the a* values found based on the image data of scalps are displayed according to age groups (each by 5 years) to be ranked into a plurality of groups.

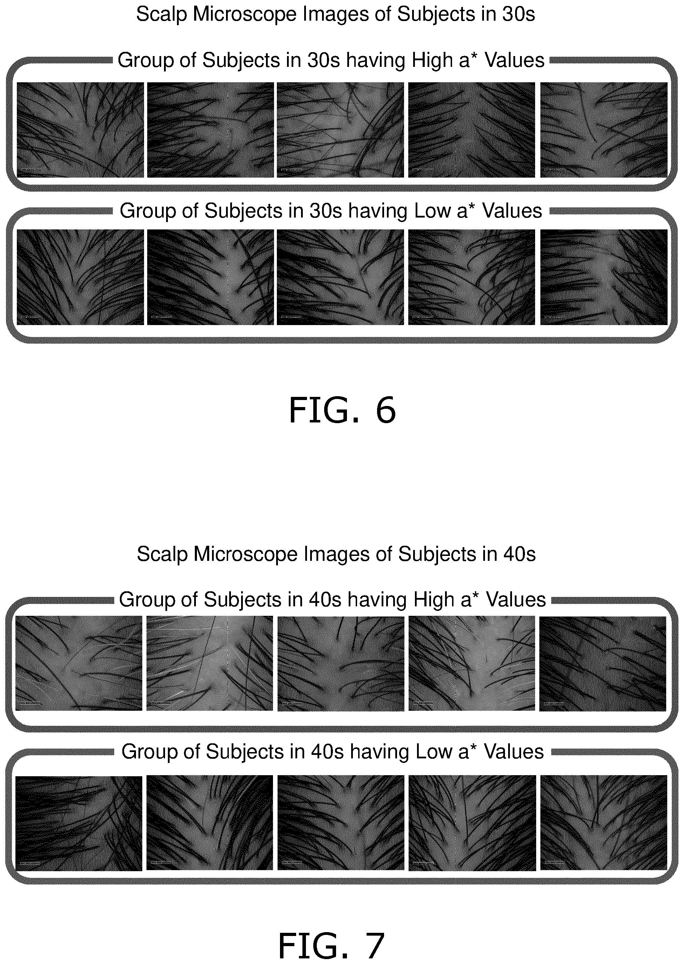

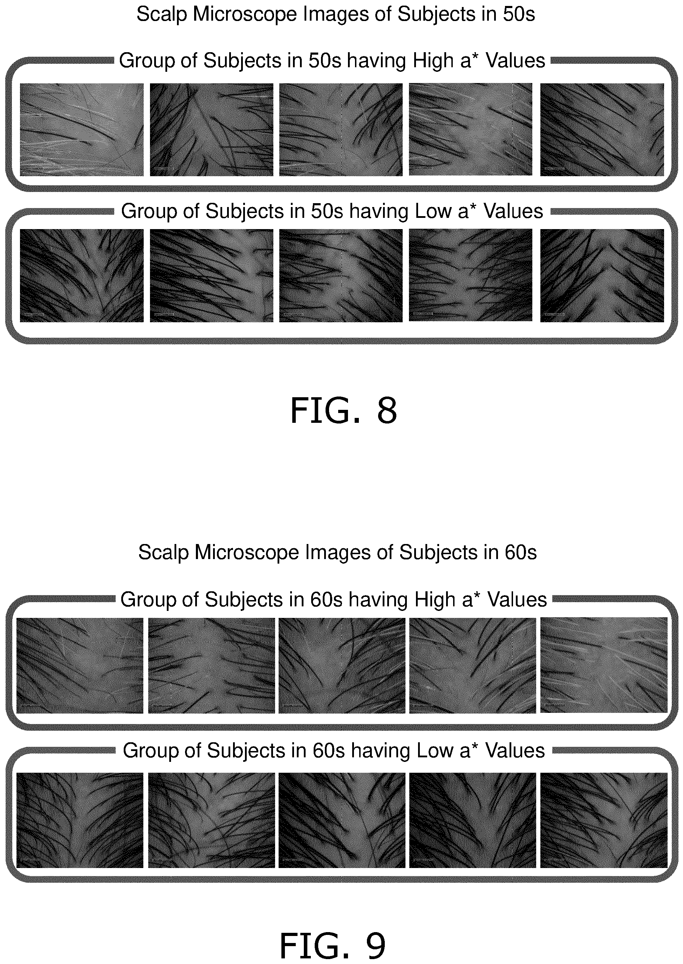

Although data of a plurality of age groups is displayed together in the form of a list in Figures 3 and 4, image data of a group having large a* values (of, for example, top 30%) and a group having low a* values (of, for example, bottom 30%) separately illustrated adjacently to each other as illustrated in Figures 5 to 9 may be prepared with respect to each age group, from which data corresponding to the age of a subject may be selected for use. It is noted that Figures 5 to 9 are created based on the image data of the scalps of the respective age groups of the 281 subjects collected for creating the table of Figure 3.

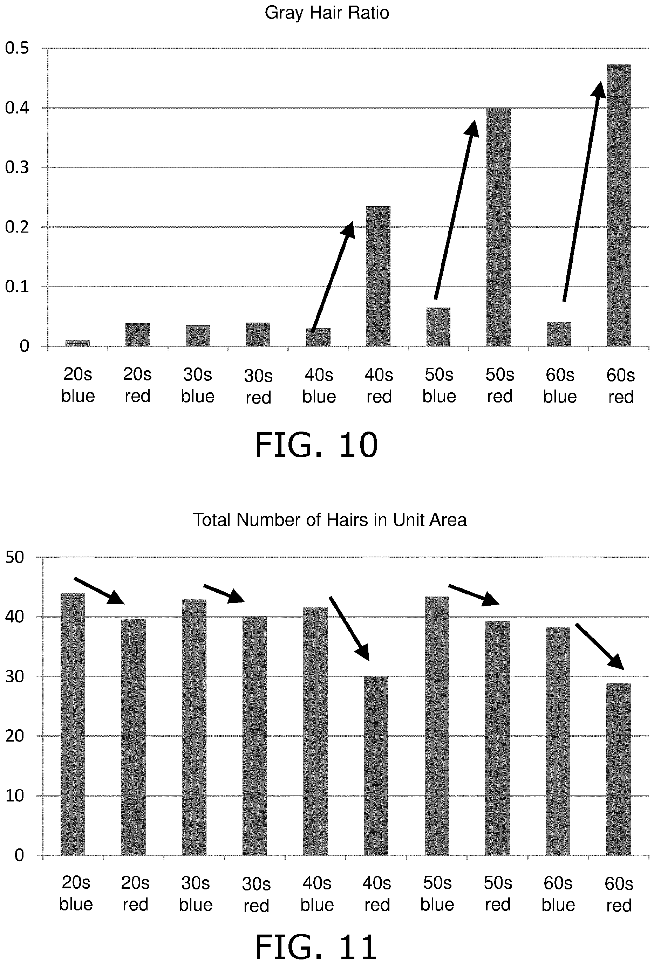

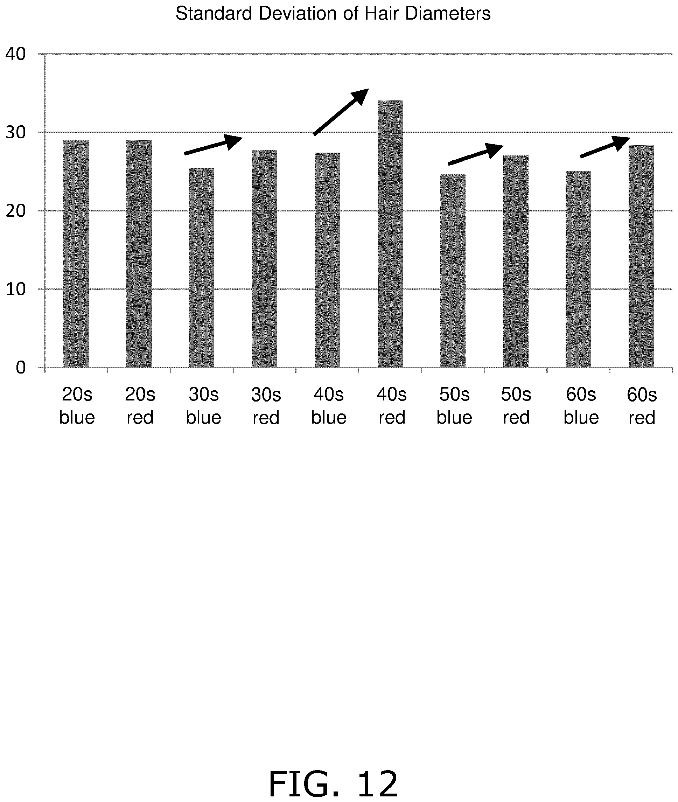

A graph of the relationship between the scalp redness and the gray hair ratio of the respective age groups obtained based on the data of the scalp images of the 281 subjects of the respective age groups collected for creating the table of Figure 3 is illustrated in Figure 10, a graph of the relationship between the scalp redness and the total number of hairs in a unit area of the respective age groups is illustrated in Figure 11, and a graph of the relationship between the scalp redness and the standard deviation of hair diameters of the respective age groups is illustrated in Figure 12. In Figures 10 to 12, "red" corresponds to the group having high a* values of the scalp (for example, top 30%), and "blue" corresponds to the group having low a* values of the scalp (for example, bottom 30%).

It is understood from Figure 10 that as the degree of scalp redness is higher, the gray hair ratio is higher in all the age groups, and in particular, a person having a high degree of scalp redness tends to have a gray hair ratio rapidly increasing when she becomes older than 40 years (see arrows illustrated in Figure 10).

Besides, it is understood from Figure 11 that a person having a higher degree of scalp redness tends to have a smaller total number of hairs in a unit area in all the age groups (see arrows illustrated in Figure 10).

It is understood from Figure 12 that a person having a higher degree of scalp redness tends to have a larger standard deviation of hair diameters and tends to easily have variation in hair diameters in the age groups excluding the age group of 20s in which no difference is found (see arrows illustrated in Figure 12).

In this manner, it is revealed, from the contents of Table 1 and Figures 1, 2 and 10 to 12, that as the abundance ratio of bacteria of the genus Corynebacterium in the scalp flora is higher, the degree of the scalp redness tends to be higher, the gray hair ratio tends to be higher, the total number of hairs in a unit area tends to be smaller and the hair diameter tends to be more easily varied.

### CITATION LIST

### PATENT LITERATURE

Patent Literature 1: Japanese Patent Laid-Open No. 2009-172023

## Claims

1. An evaluation method for performing evaluation of at least any one of scalp redness, an index related to the redness, a largeness in number of gray hairs, a smallness in number of hairs and a largeness of variation in hair thickness of an evaluatee based on information on bacteria of the genus Corynebacterium present in a scalp flora of the evaluatee, or
for performing evaluation of information on bacteria of the genus Corynebacterium present in a scalp flora of an evaluatee based on at least any one of scalp redness, an index related to the redness, a largeness in number of gray hairs, a smallness in number of hairs and a largeness of variation in hair thickness of the evaluatee.

2. The evaluation method according to claim 1, wherein the information on bacteria of the genus Corynebacterium present in the scalp flora of the evaluatee includes an abundance ratio of bacteria of the genus Corynebacterium in the scalp flora of the evaluatee.

3. The evaluation method according to claim 2, wherein the evaluation is performed by using a comparison criterion for an abundance ratio of bacteria of the genus Corynebacterium in a scalp flora.

4. The evaluation method according to claim 3, wherein the evaluation is performed by using a comparison criterion, according to an age of the evaluatee, for an abundance ratio of bacteria of the genus Corynebacterium in a scalp flora.

5. A selection method for a scalp/hair cosmetic for selecting a scalp/hair cosmetic to be recommended to the evaluatee according to an evaluation result obtained by the evaluation method according to any one of claims 1 to 4.

6. The selection method for a scalp/hair cosmetic according to claim 5, wherein a scalp/hair cosmetic containing an anti-inflammatory agent is selected based on the evaluation result.

7. An evaluation support tool for supporting evaluation of at least any one of scalp redness, an index related to the redness, a largeness in number of gray hairs, a smallness in number of hairs and a largeness of variation in hair thickness of an evaluatee based on information on bacteria of the genus Corynebacterium present in a scalp flora of the evaluatee, or
for supporting evaluation of information on bacteria of the genus Corynebacterium present in a scalp flora of an evaluatee based on at least any one of scalp redness, an index related to the redness, a largeness in number of gray hairs, a smallness in number of hairs and a largeness of variation in hair thickness of the evaluatee,
wherein a comparison criterion for an abundance ratio of bacteria of the genus Corynebacterium in a scalp flora is displayed.

8. A screening method for a scalp/hair cosmetic, comprising the steps of:
treating a scalp and/or hair of an evaluatee with a scalp/hair cosmetic at least once; and
comparing abundance ratios of bacteria of the genus Corynebacterium in a scalp flora of the evaluatee before and after the step of treating a scalp and/or hair.

9. A screening method for a candidate substance for an active ingredient, comprising the steps of:
treating a scalp and/or hair of an evaluatee with a candidate substance of an active ingredient at least once; and
comparing abundance ratios of bacteria of the genus Corynebacterium in a scalp flora of the evaluatee before and after the step of treating a scalp and/or hair.

10. A screening method for selecting an evaluatee, comprising the steps of:
checking an abundance ratio of bacteria of the genus Corynebacterium in a scalp flora of an evaluatee; and
comparing the abundance ratio of the evaluatee with a comparison criterion for an abundance ratio of bacteria of the genus Corynebacterium in a scalp flora.

11. An evaluation method for performing evaluation of at least any one of a largeness in number of gray hairs, a smallness in number of hairs, a largeness of variation in hair thickness and a thin hair ratio according to scalp redness or an index related to the redness of an evaluatee.

12. The evaluation method according to claim 11, wherein the evaluation is performed by using information corresponding to a comparison criterion, according to an age of the evaluatee, for scalp redness or an index related to the redness.

13. A selection method for a hair treatment agent for selecting a hair treatment agent to be recommended to the evaluatee according to an evaluation result obtained by the evaluation method according to claim 11 or 12.

14. The selection method for a hair treatment agent according to claim 13, wherein a hair treatment agent containing an anti-inflammatory agent is selected based on the evaluation result.

15. An evaluation support tool for supporting evaluation of at least any one of a largeness in number of gray hairs, a smallness in number of hairs, a largeness of variation in hair thickness and a thin hair ratio, wherein information corresponding to a comparison criterion to be compared with scalp redness or an index related to the redness of an evaluatee is displayed.
